# EUROPEAN PATENT SPECIFICATION

(11) **EP 1 242 060 B1**
(45) Date of publication and mention of the grant of the patent: **03.05.2006**
(21) Application number: 00955687.9
(22) Date of filing: 17.08.2000
(51) Int. Cl.: A61K 31/00, A61K 39/395, A61K 49/00, C07K 2/00, C07K 5/00, C07K 16/28, G01N 33/50, A61P 35/04

(54) **TREATMENT OF METASTATIC DISEASE**
BEHANDLUNG VON METASTATISCHER KRANKHEIT
TRAITEMENT DE MALADIES METASTATIQUES

(30) Priority: 17.08.1999 US 149258 P
(43) Date of publication of application: 25.09.2002
(73) Proprietor: Purdue Research Foundation, West Lafayette, IN 47906 (US); GLAXO GROUP LIMITED, Greenford, Middlesex UB6 0NN (GB); UNIVERSITY OF NORTH CAROLINA AT CHAPEL HILL, Chapel Hill, NC 27599-4105 (US)
(72) Inventor: KINCH, Michael, Scott, Lafayette, IN 47905 (US); KILPATRICK, Katherine E., Chapel Hill, NC 27516 (US); ZANTEK, Nicole, D., Silver Springs, Maryland 20905 (US); HEIN, Patrick, W., San Francisco, CA 94129 (US)
(74) Representative: Vossius & Partner
(86) International application number: PCT/US2000/022670
(87) International publication number: WO 2001/012172

(56) References cited:
- WO-A-00/30673
- DODGE ZANTEK N ET AL: "IDENTIFICATION OF AN ADHESION-ASSOCIATED TYROSINE KINASE THAT IS TIGHTLY REGULATED IN BREAST CANCER" MOLECULAR BIOLOGY OF THE CELL, BETHESDA, MD, US, vol. 8, no. SUPPL, November 1997 (1997-11), page 134A, XP000979706 ISSN: 1059-1524
- ZANTEK N D ET AL: "EPITHELIAL CELL KINASE (ECK/EPHA2) REGULATION IN BREAST CANCER" MOLECULAR BIOLOGY OF THE CELL, BETHESDA, MD, US, vol. 9, no. SUPPL, November 1998 (1998-11), page 134A, XP000980380 ISSN: 1059-1524
- ZANTEK N D ET AL: "REGULATION OF THE EPHA2 RECEPTOR TYROSINE KINASE BY ESTROGEN AND MYC" PROCEEDINGS OF THE ANNUAL MEETING OF THE AMERICAN ASSOCIATION FOR CANCER RESEARCH, NEW YORK, NY, US, vol. 40, March 1999 (1999-03), page 687, XP000978765 ISSN: 0197-016X
- EASTY DAVID J ET AL: "Protein B61 as a New Growth Factor: Expression of B61 and Up-Regulation of Its Receptor Epithelial Cell Kinase during Melanoma Progression." CANCER RESEARCH, vol. 55, no. 12, 1995, pages 2528-2532, XP001147293 ISSN: 0008-5472
- KINCH M.S. ET AL.: 'Identification of tyrosine phosphorylated adhesion proteins in human cancer cells' HYBRIDOMA vol. 17, no. 3, 1998, pages 227 - 235, XP002936218
- ZANTEK N.C. ET AL.: 'E-cadherin regulates the function of the EphA2 receptor tyrosine kinase' CELL GROWTH & DIFFERENTIATION vol. 10, September 1999, pages 629 - 638, XP002936217
- NAKAMOTO M.: 'Eph receptors and ephrins' INTERNATIONAL JOURNAL OF BIOCHEMISTRY & CELL BIOLOGY vol. 32, 1999, pages 7 - 12, XP002936216
- HUAI J. ET AL.: 'Investigation of a possible receptor function of ephrinA ligands' EUROPEAN JOURNAL OF NEUROSCIENCE vol. 12, 2000, page 179, ABSTRACT NO. 07910, XP002936215
- WALKER-DANIELS J. ET AL.: 'Overexpression of the EphA2 tyrosine kinase in prostate cancer' THE PROSTATE vol. 41, 1999, pages 275 - 280, XP002936219
- LINDBERG R.A. ET AL.: 'cDNA cloning and characterization of ECK, an epithelial cell receptor protein-tyrosine kinase in the EPH/ELK family of protein kinases' MOLECULAR AND CELLULAR BIOLOGY vol. 10, no. 12, December 1990, pages 6316 - 6324, XP002936214

## Description

### Field of the Invention

The present invention relates to the use of an epithelial cell tyrosine kinase that is overexpressed in metastatic tumor cells as the target for the treatment of metastatic disease. Most particularly, this invention relates to the use of compounds that interact with and alter expression of the epithelial ceiltyrosinekinase.

### Background and Summary of the Invention

Cancer is a disease of aberrant signal transduction. The most dangerous forms of cancer are malignant cells that metastasize to distant sites in a body: Metastatic cells have acquired the ability to break away from the primary tumor, translocate to distant sites, and colonize distant and foreign microenvironments. Cancer cell metastasis requires cellular capacity to 1) detach from a primary tumor, 2) migrate and invade through local tissues, 3) translocate to distant sites in the body (via lymph or blood), 4) colonize a foreign site, and 5) grow and survive in this foreign environment. All of these behaviors are linked to cell adhesions. Cell adhesions control the physical interactions of cells with their microenvironment Cell adhesions also initiate signals that dictate tumor cell growth, death, and differentiation. At the cellular level, metastatic cells have overcome restraints upon cell growth and nilgration that result from physical linkages and signals conveyed by cell-cell contacts. Malignant cells often have increased interactions with surrounding extracellular matrix (ECM) proteins that provide linkages and signals that promote several aspects of metastasis.

Levels of protein tyrosine phosphorylation regulate a balance between cell-cell and cell-ECM adhesions in epithelial cells. Elevated tyrosine kinase activity weakens cell-cell contacts and promotes ECM adhesions. Alteration in levels of tyrosine phosphorylation is believed to be important for tumor cell invasiveness. Tyrosine phosphorylation is controlled by cell membrane tyrosine kinases, and increased expression of tyrosine kinases is known to occur in metastatic cancer cells.

EphA2 is a 130 kDa receptor tyrosine kinase that is expressed on adult epithelia. A member of the Eph family of tyrosine kinases known as Ephrins, EphA2 is a transmembrane receptor tyrosine kinase with a cell-bound ligand. EphA2 expression has been found to be altered in many metastatic cells, including lung, breast, colon, and prostate tumors. Additionally, the distribution and/or phosphorylation of EphA2 is altered in metastatic cells. Moreover, cells that have been transformed to overexpress EphA2 demonstrate malignant growth, and stimulation of EphA2 is sufficient to reverse malignant growth and invasiveness. EphA2 is a powerful oncoprotein. The present invention is directed to uses as described herein that target EphA2 for the treatment of metastatic cancers.

One approach to cancer therapy is the administration of preformed antibodies to predetermined tumor antigens. This process is known as passive antibody treatment An example of passive antibody treatment is the use of Herceptin® for the treatment of breast cancer. Herceptin® is a humanized form of a murine monoclonal antibody specific for the extracellular domain of Her2/Neu. The basis for treatment with Herceptin® is that 25-30% of metastatic breast cancers overexpress the Her2/Neu receptor tyrosine kinase. Herceptin® has been well tolerated in clinical trials and shows much promise for the maintenance and regression of metastatic breast cancer.

Effective passive immunotherapy for treatment of tumors requires isolation and preparation of an antibody that: 1) targets an antigen that is overexpressed in metastatic tumors; 2) targets an extracellular epitope of said antigen; 3) is not cross-reactive with any other antigen in a patient's circulation; and 4) exhibits tumoricidal or tumoristatic activity.

In a preferred embodiment, this invention relates to the selection and use of antibodies that are specific to an extracellular epitope of EphA2. The uses of this invention include the preparation, selection, and use of EphA2 specific antibodies for cancer therapy.

Another approach to cancer treatment is to use agonists to stimulate expression. For example, EphrinA1-F_{c}, the extracellular domain of ephrinA1 linked to immunoglobulin heavy chain (see Miao, H., et al., EphA2 kinase associates with focal adhesion kinase and upon activation, inhibits integrin-mediated cell adhesion and migration, Nature Cell Biol 2, 62-69 (2000)), can be used to increase the phosphotyrosine content of EphA2. Thus, in another preferred embodiment, this invention relates to the use of agonists to alter the expression of EphA2 in metastatic cells as described herein.
Thus, this invention is directed to the use of agonists to alter the expression of EphA2 as described herein. EphA2 may be targeted by use of artificial or hybrid forms of the protein, protein inhibitors, antisense oligonucleotides, or small molecule inhibitors. Also, while a preferred embodiment is directed to use of monoclonal antibodies, polyclonal, artificial, and hybrid antibodies are known in the art. It should be understood that use of techniques known in the art to target EphA2 are within the scope of this invention.
On aspect of this invention is the use of an EphA2 agonist selected from (i) a compound comprising an extracellular domain of EphrinA1 and (ii) an antibody that is specific to an extracellular epitope of EphA2 having at least one biological activity for reducing metastatic invasion or proliferation of a tumor, for reducing metastasis of metastatic cells, for impeding proliferation of metastatic cells, for increasing the phosphotyrosine content of EphA2 in metastatic cells, and/or for reducing the invasiveness of metastatic cells compared to untreated metastatic cells for the preparation of a pharmaceutical composition for treatment of a mammalian metastatic tumor or cancer including a population of cells which overexpresses tyrosine kinase EphA2.

A further aspect of the present invention is the use of an EphA2 agonist selected from (i) a compound comprising an extracellular domain of EphrinA1 and (ii) an antibody that is specific to an extracellular epitope of EphA2 for the manufacture of a medicament for the treatment of a metastatic tumor comprising a population of cells that overexpress EphA2.

A still further aspect of the present invention is the use of an EphA2 agonist selected from (i) a compound comprising an extracellular domain of EphrinA1 and (ii) an antibody that is specific to an extracellular epitope of EphA2 for the manufacture of a medicament for the treatment of a patient having a metastatic or potentially metastatic cancer comprising a population of cells that overexpress EphA2.
Accordingly, a patient can be treated with the uses as described herein having a metastatic tumor which overexpresses EphA2. The use comprises administering to the patient a therapeutic amount of compound as described herein that binds to an extracellular epitope of EphA2. In a preferred embodiment, the compound is an antibody.

Also described is a method for producing antibodies which inhibit metastatic tumor proliferation by specifically binding to an extracellular epitope of EphA2. This method includes injecting tyrosine phosphorylated proteins into the lymph nodes of a mammal, harvesting the lymph nodes, fusing the lymph node cells with myeloma cells to form hybridomas, and selecting hybridomas which produce antibodies specific for EphA2.

Additional features of the present invention will become apparent to those skilled in the art upon consideration of the following detailed description of preferred embodiments exemplifying the best mode of carrying out the invention as presently perceived.

### Brief Description of the Drawings

Fig. 1 is an overview of the RIMMS procedure, through which the antibodies of this invention are generated;
Fig. 2A-C show a series of western blots showing EphA2 expression in human cell lines;

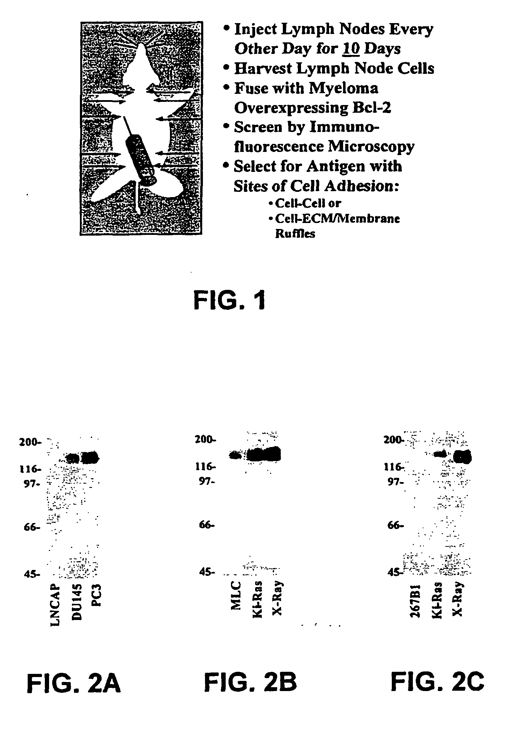

Fig. 2A is a western blot showing EphA2 expression in various human prostate cancer cell lines;
Fig. 2B is similar to Fig. 2A, except showing EphA2 expression in a human prostatic epithelial cell line and expression in the cell line after transformation by oncogenic K-Ras or X-irradiation;
Fig. 2C is similar to Fig. 2B, except showing EphA2 expression in another human prostatic epithelial cell line and expression in the cell line after transformation by oncogenic K-Ras or X-irradiation;

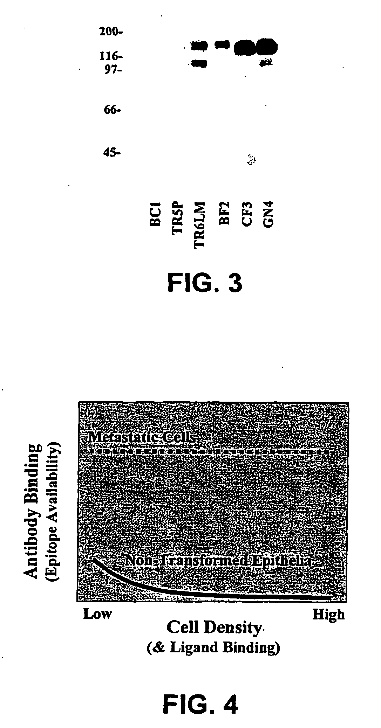

Fig. 3 is similar to Fig. 2, except showing EphA2 expression in canine prostatic cancer cells; and
Fig. 4 shows predicted antibody binding plotted against cell density in a screening procedure for antibodies which are specific for an extracellular epitope of EphA2.

### Detailed Description of the Invention

EphA2 is expressed differently in normal and metastatic cells. In normal breast and prostate epithelial cells, EphA2 is enriched in within sites of cell adhesion. Conversely, in metastatic prostate cells EphA2 is diffusely distributed, and in metastatic breast cancer cells EphA2 is redistributed into the membrane ruffles. EphA2 expression is also known to be altered in lung and colon malignancies, and it is believed that EphA2 altered expression occurs in other types of metastasis, particularly epithelial malignancies. Thus, techniques designed to alter EphA2 expression can be exploited to diagnose and treat metastatic disease.

In a preferred embodiment, antibodies specific for tyrosine phosphorylated proteins in cancer cells have been isolated and used to target cancer cells in passive immunotherapy. This approach is based upon the fact that many tyrosine kinases; e.g., Her2/Neu, are expressed by oncogenes and are therefore overexpressed in cancer cells. The present invention is directed to the use of antibodies capable of recognition of and specific binding to extracellular epitopes of the tyrosine kinase EphA2. The antibodies are produced by selected hybridomas, themselves the product of fusion of myeloma cells with lymph node cells harvested from animals subjected to a specific inoculation protocol designed for increased sensitivity and diversity of responding hybridomas.

To produce these hybridomas, tyrosine phosphorylated proteins from Ras-transformed human epithelial cells were isolated by affinity chromatography using existing phosphotyrosine specific antibodies. The tyrosine phosphorylated proteins are then used as an immunogen for producing monoclonal antibodies according to the procedure illustrated in Fig. 1. Low-dose amounts of tyrosine phosphorylated proteins are injected proximal to lymph nodes of a mammal, every other day, over a ten day period (the RIMMS strategy). B cells from engorged lymph nodes are then isolated and fused with Bcl-2-overexpressing myeloma cells, to minimize apoptosis after fusion. This method results in increased diversity, specificity, and cost-effectiveness of hybridoma production. The hybridomas are screened to identify those hybridomas producing antibodies that distinguish malignant from normal cancer cells.

Hybridomas producing antibodies specific to EphA2 have been selected. Use of the RIMMS technique has resulted in the production of a multiplicity of hybridomas producing monoclonal antibodies that specifically bind EphA2. To date, at least 450 hybridomas have been identified which produce antibodies capable of distinguishing malignant from normal cancer cells. Of the first four such hybridomas to be characterized, two recognize independent epitopes on EphA2. The first, D7, produces an antibody recognizing an intracellular epitope. The second, B2D6, produces an antibody that specifically binds an extracellular epitope of EphA2, a characteristic that enables its effective use for the diagnosis and treatment of selected metastatic tumors.

While the RIMMS strategy has proven to be valuable in the production of EphA2 specific antibodies, other techniques are known in the art for producing antibodies to a specific antigen, and these techniques are within the scope of this invention.

It is known in the art to use antibodies to detect the presence or overexpression of a specific protein. Because EphA2 is overexpressed in metastatic cells, EphA2-specific antibodies of this invention may be used to detect this overexpression and, thus, to detect metastatic disease. Such techniques include but are not limited to western blotting, precipitation, agglutination, and ELISA assays. These techniques are well known in the art. Also, the extracellular epitope specificity of EphA2-specific antibodies of this invention can be exploited to detect changes in EphA2 localization which are associated with metastasis. In normal breast and prostate epithelial cells, EphA2 is enriched in within sites of cell adhesion, whereas in metastatic cells, EphA2 distribution is altered. In metastatic prostate cells EphA2 is diffusely distributed, and in metastatic breast cancer cells EphA2 is redistributed into the membrane ruffles. EphA2 expression is also known to be altered in lung and colon malignancies, and it is believed that EphA2 altered expression occurs in other types of metastasis, particularly epithelial malignancies Techniques such as immunohistological staining or immunofluorescent microscopy are well known in the art and may be used to visualize EphA2 distribution. See, for example, U.S. Patent No. 5,514,554. In order to detect overexpression or altered distribution of EphA2, the EphA2-specific antibodies may be labeled covalently or non-covalently with any of a number of known detectable labels, such fluorescent or radioactive substances, as is known in the art. Alternatively, a secondary antibody specific for the antibodies of this invention is labeled with a known detectable label and used to detect the EphA2-specific antibodies in the above techniques. Thus, the antibodies of this invention provide methods to detect metastatic transformation.
The present invention also employs antibodies specific for an extracellular epitope of EphA2 ii therapeutic uses as described herein. When used for in vivo therapy, a pharmaceutical composition as described in connection with the uses of the present invention is to be administered to a patient It comprises EphA2-specific antibodies in therapeutically effective amounts in a pharmaceutically acceptable carrier. In a preferred embodiment, the EphA2-specific antibodies have been "humanized." Humanized antibodies include "chimeric antibodies" made by splicing genes from a mouse (or other mammal) antibody of appropriate antigen specificity together with genes from a human antibody molecule of appropriate biological activity. Such techniques are known in the art. See, for example, U.S. Patent No. 5,811,098. In addition to antibodies, natural or artificial ligands, peptides, anti-sense, ATP analogues, or other small molecules capable of specifically targeting EphA2 may be employed.

An example of another way to target EphA2 is the use of ephrins to activate or inhibit EphA2. For example, EphrinA1-F_{c}, the extracellular domain of ephrinA1 linked to immunoglobulin heavy chain, increases the phosphotyrosine content of EphA2. EphrinA1-F_{c} reverses the malignant behavior of EphA7 transformed cells. Thus, another preferred embodiment of this invention is the use of ephrin or a hybrid form of ephrin for the preparation of a pharmaceutical composition as described herein which is to be administered in a therapeutic amount.

Therapeutic amounts are amounts which eliminate orreduce the patimes-mmor burden, or which prevent or reduce the proliferation of metastatic cells. The dosage will depend on many parameters, including the nature of the tumor, patient history, patient condition, the possible co-use of other oncolytic agents, and methods of administration. Methods of administration include injection (eg., parenteral, subcutaneous, intravenous, etc.) for which the antibodies are provided in a nontoxic pharmaceutically acceptable carrier such as water, saline, Ringer's solution, dextrose solution, 5% human serum albumin, fixed oils, ethyl oleate, or liposomes. Typical dosages may range from about 0.01 to about 20 mg/kg, and more particularly from about 0.1 to about 10 mg/kg. Other methods of administration include oral and transdermal. Acceptable carriers for oral ingestion in accordance with the present invention can be formulated using art-recognized techniques into pharmaceutically acceptable liquid carriers or in combination with pharmaceutically acceptable solid carriers in the form of tablets, capsules, caplets, or gel-seals. Other effective methods of administration and dosages may be determined by routine experimentation and are within the scope of this invention.

Therapeutic uses employing EphA2-specific antibodies may be combined with chemotherapy, surgery, and radiation therapy, depending on type of the tumor, patient condition, other health issues, and a variety of factors. The methods may also include immunoconjugates for targeted immunotoxin-mediated therapy, wherein antibodies of this invention are covalently or non-convalently conjugated to various cytotoxic agents, further enhancing toxicity to targeted cells. See, for example, U.S. Patent No. 5,872,223. Such agents, including various bacterial toxins (e.g., *Pseudomonas* exotoxin), ricin A-chain, daunorubicin, methotrexate, and ribosome inhibitors (e.g., trichosantin). Also, the antibodies of this invention may be labeled with alpha, beta, or Auger electron emitters, resulting in immunoconjugates for targeted radiotherapy.

Thus, EphA2-specific antibodies may be used in a variety of uses for treating metastatic disease.

### EXAMPLE 1.

### Charaeterization of EphA2 Expression in Metastatic Cells

Following the RIMMS strategy using tyrosine phosphorylated proteins from Ras-transformed human epithelial cells, hybridomas were screened, and an antibody specific for EphA2 has been isolated. This antibody was used to assess the levels of EphA2 expression in nontransformed prostatic epithelial cells and prostatic tumor cells. Low levels of EphA2 expression were found in non-transibaned prostatic epithelial cells, but this EphA2 expression was enriched within sites of cell-cell contact and interacted with cell-bound ligand. Compared to non-transformed cells, two features distinguish EphA2 in metastatic prostate cancer cells: 1) EphA2 is overexpressed; 2) EphA2 is diffusely distributed and does not appear to interact with ligand. To confirm these data, western blots were performed using the EphA2 specific antibodies. EphA2 overexpression in human prostate cancer cells (LNCAP, DU145, PC3) directly correlates with their invasiveness *in vitro* and *in vivo.* Of the three lines tested, LNCAP is the least aggressive, DU145 is more aggressive, and PC3 is the most aggressive. As seen in Fig. 2, DU145 cells exhibit higher levels of EphA2 expression than LNCAP, and PC3 cells exhibit even higher levels of EphA2 expression. Similarly, as shown in Figs. 2B and 2C, EphA2 expression is elevated in variants of human prostatic epithelial cells transformed by oncogenic K-Ras or X-irradiation. The three lanes in Fig. 2B show "normal" MCL prostatic epithelial cells, and K-Ras and X-ray transformed cell lines derived therefrom. Similarly, the three lanes of Fig. 2C show "normal" 267B1 prostatic epithelial cells, and K-Ras and X-ray transformed cell lines derived therefrom. As seen in Figs. 2B and 2C, the transformed cells all exhibited elevated EphA2 levels. Fig. 3 shows similar western blots, except using prostate cancer cell lines from dogs. As shown in Fig. 3, consistent with the results from human cells, EphA2 is overexpressed in metastatic prostatic carcinoma cells derived from dogs with spontaneous prostate cancer.

The metastatic prostate cell lines can be subdivided into three categories: 1) cells derived from primary prostate tumors; 2) cells derived from metastases that are poorly metastatic *in vivo;* 3) cells derived from metastases that are highly metastatic *in vivo.* The western blots using EphA2-specific antibodies have revealed that EphA2 expression is elevated in all cells derived from metastases, with highest EphA2 expression in cells that retain metastatic potential *in vivo* (as assessed using athymic mouse models). Interestingly, B2D6 studies have shown that EphA2 is overexpressed in cells from prostate cancer metastases compared to lines established from the primary tumor of same patient. Taken together, these results all reveal EphA2 overexpression in metastatic prostate tumor cells.

Similar EphA2 expression patterns have been found with breast cancer cells. In normal mammary epithelial cells, EphA2 is enriched within the cell-cell junctions. By contrast, non-metastatic breast cancer cells do not express EphA2, while metastatic breast cancer cells overexpress EphA2. In metastatic breast cancer cells, EphA2 is redistributed into the membrane ruffles and, thus, is available for antibody binding.

### EXAMPLE 2

### In Vitro Targeting of Metastatic Cells

EphA2 overexpression renders metastatic cells susceptible to antibody-mediated selected killing with the present antibodies specific for an extracellular epitope of EphA2. While normal cells express EphA2, it is believed that ligand binding or clustering within sites of cell-cell contact occludes extracellular epitopes in normal cells and renders them inaccessible to antibodies specific for an extracellular epitope of EphA2. The tumor selectivity of the antibodies of the present invention is believed to rival or exceed that of Herceptin® for targeting metastatic cancer.

EphA2 overexpression provides a basis for targeting metastatic cancer cells with EphA2-specific antibodies. Antibodies specific for an extracellular epitope of EphA2, such as those produced by hybridoma B2D6, may be used to alter selectively (versus normal cells) the proliferative or invasive behaviors of metastatic cancer cells. In both metastasis-derived and laboratory-induced transformation, EphA2 overexpression correlates with invasiveness, whereas non-invasive cells have lower levels of EphA2 expression.

To measure the effect of B2D6 on cell growth, cells are incubated with purified B2D6, and cell proliferation is measured by counting cells microscopically (using a hemacytometer) and by measuring DNA synthesis. For example, normal growth media is supplemented with B2D6 and BrdU, and BrdU incorporation is measured over the following four hours. To measure the effects of B2D6 over longer times, samples are counted at 24 hour intervals, with BrdU added to the culture media for the final four hours of incubation. As a third measure of cell growth, the effect of B2D6 on the growth of metastatic cells in soft agar is determined. Soft agar plating assays are used, wherein 2x10⁴ metastatic cells are plated atop agar, in the presence or absence of B2D6 (0-10 nM), and colony growth is evaluated at three-day intervals thereafter.

It is believed that B2D6 decreases the growth of metastatic cells. Preliminary results reveal that B2D6 aggregates EphA2 and blocks about 50% of growth of metastatic breast cancer cells (which also overexpress EphA2) over the first four hours of incubation. Although EphA2 is not tyrosine phosphorylated in metastatic breast cancer cells, tyrosine phosphorylation is restored these B2D6 treated cells. Thus, B2D6 is believed to restore normal EphA2 function.

Additional studies with prostate cancer cells are being performed to determine if longer incubations with B2D6 further inhibit metastatic cell growth. Non-transformed epithelial cells express some EphA2, albeit much less than metastatic cells, and some toxicity to non-transformed cells is possible. The minimum effective and maximum non-toxic dosage levels of antibodies in accordance with one aspect of this invention can be identified by routine experimentation, but preferably, typical doses will range from about 0.1 to about 20 mg/kg of patient body weight. The preferred dose will depend on many parameters, including the nature of the tumor, patient history, patient condition, the possible co-use of other oncolytic agents, and methods of administration. Antibody levels that best discriminate between normal and metastatic cells will be used in treatment of metastatic tumors overexpressing EphA2 proteins.

### EXAMPLE 3

### In Vitro Antibody Mediated Cytotoxicity

Preliminary results demonstrate that EphA2 antibodies impede metastatic cell growth. To measure antibody-directed cytotoxicity, preferably, nonradioactive versions of ⁵¹Cr-release assays using target cells (normal or metastatic prostatic epithelial cells) labeled with europium chloride (EuCl₃) and diethylenetriamine-pentaacetic acid (DTPA) are performed. After washing away unincorporated Eu³⁺, naphthoyltrifluoroacetone (NTA) and trioctylphosphine oxide are incubated with the cytolydc agents and assay supernatants. The luminescence of resultant ternary complex (Eu³⁺/NTA/trioctylphosphine oxide) is measured using a fluorescence microplate reader. The sensitivity of this Eu³⁺-release assay for complement-mediated cytolysis has been reported to be fivefold better than ⁵¹Cr-release assays. To determine specific lysis, parallel samples are hyponically lysed by adding distilled water. Untreated samples and isotype matched antibodies serve as negative controls.

To model complement-mediated death *in vitro,* cells are labeled with B2D6 and exposed to sera that has not been heat-inactivated. For antibody dependent cellular cytotoxicity ("ADCC"), both control and B2D6-treated prostate cells are incubated with peripheral blood mononuclear cells (PBMC; 10:1 E/T ratio).

It is believed that complement and ADCC both promote specific killing of the metastases. Antibody dose can be varied in order to establish LD₅₀ measurements for metastatic and normal cells. Antibody concentrations that maximize specific killing of metastatic cells (PC3, 267-Ras, 267-X) while minimizing the death to non-transformed prostatic epithelia (267, MLC) are determined by routine experimentation. Also, ADCC can be combined with complement to further enhance tumor killing by treating the samples with tumor necrosis factor (TNF). There is evidence that TNF potentiates the killing of tumor cells by EGFR and Her2-specific antibodies. It is expected that humanizing the antibodies of this invention would provide for better complement or ADCC results.

It is believed that B2D6 kills tumor cells through complement cascade or ADCC. However, covalent or non-covalent conjugation of the present antibodies to art-recognized cytotoxic agents can further enhance toxicity to targeted cells. Examples of toxins appropriate for immunoconjugation include *Pseudomonas* exotoxin or ricin A-chain.

### EXAMPLE 4

### In Vivo targeting of Metastatic Cells

The present EphA2 antibodies, particularly those produced by hybridoma B2D6 are effective in blocking the growth and invasiveness of prostate cancer cells *in vivo.* The efficacy of B2D6 in blocking the growth of primary prostate tumors using subcutaneous implantation of PC3 tumor cells in mice is determined by use of subcutaneous models. The primary advantages of subcutaneous models are the ease of implantation and subsequent monitoring of tumor size. 5x10⁵ PC3 cells are inoculated subcutaneously into the right craniolateral thorax (axilla) using aseptic technique. Tumors are measured every 3-4 days using vernier calipers until they reach a volume of 0.2-0.3 cm³. At that time, the mice are divided into four groups (8-10 animals each): Group 1 (vehicle control), Groups 2-4 are treated with 0.1, 1.0, or 10 mg/kg B2D6, administered intraperitoneally, twice a week. The mice are then monitored every 3 days to measure tumor volume (with vernier calipers), body weight, and life span. After no greater than 60 days past implantation, the animals are sacrificed and postmortem evaluations of tumorigenesis, including measurement and weight of implanted tumors and proximal lymph nodes, macroscopic evaluation of soft tissues for tumors (lymph nodes and lung), and formalin fixation of the primary tumor and tissues, are performed. The tissues are evaluated by immunohistochemistry using D7 (another EphA2 specific antibody that is amenable to immunohistochemistry) to determine the level of EphA2 expression in the tumors. In particular, tumor cells that escape B2D6 treatment are studied to determine whether they have low levels of EphA2 expression. Also, EphA2 expression in the individual animals is correlated with tumor invasiveness.

As target-negative controls for specificity, parallel studies are performed using DU145 cells, which express very low levels of EphA2. Whereas the growth of PC3 tumors are believed to be sensitive to B2D6, tumors caused by DU145 are believed be insensitive. Statistical significance of B2D6 inhibition of tumorigenicity, overall metastatic frequency, and frequency of distant metastases is tested using computerized statistical packages (PC/SAS Ver. 6.04), with differences considered significant if p<0.05.

While subcutaneous implantation is a popular and valuable method for modeling tumor cell growth, differences between the microenvironment of the skin and prostate can cause rather dramatic differences in cell behavior. For example, PC3 cells rarely metastasize when implanted subcutaneously whereas intraprostatic implantation (orthotopic) facilitates metastasis. Thus, PC3 cells are implanted in the prostate by exposing the prostate via laparotomy and inoculating tumor cells into prostate gland using a surgical microscope. After seven days, the mice begin receiving treatment with B2D6, as described above, and the animals are sacrificed no later than 60 days after implantation (or if the animals become moribund). The tumors are palpated at 3-5 day intervals, at which time data on tumor size, animal weight, and survival are collected. Post-mortem evaluations are also performed as described above, with emphasis upon the effect of B2D6 upon metastatic potential (to lungs and regional lymph nodes). B2D6 is believed to block the primary tumor and metastatic potential of PC3 cells in a dose-dependent manner.

To minimize identification of strain or clonal-specific effects, identical analyses using other model systems can be employed. For example, the effects of B2D6 on the growth or metastasis of tumors caused by implantation of K-Ras or X-ray transformed 267B1 can be compared to the effects on MLC human prostatic epithelial cell lines.

### EXAMPLE 5

### Development of "Second Generation" EphA2-based Antibody Therapeutics

While EphA2 overexpression in metastatic prostate cancer cells provides a degree of selectivity comparable to Herceptin® in breast cancer, unique properties of EphA2 are believed to allow for even more selective targeting. In particular, EphA2 at the surface of non-transformed epithelia is tightly packed into cell-cell contacts whereas EphA2 on metastatic cells is diffusely distributed. It is thus likely that some epitopes on EphA2 are accessible in metastases but protected by ligand in normal prostatic epithelia. The EphA2 antibodies that provide the optimal discrimination between normal and metastatic prostatic epithelia are selected.

The panel of antibodies generated previously are screened for epitopes on EphA2 that are found at the cell surface. Using flow cytometry, EphA2 expression in a variety of "normal" (e.g., 267B or MLC cells) and metastatic cells (PC3 cells) are compared. For example, confluent monolayers of normal and metastatic cells are labeled with B2D6. To insure that antibodies are selected that are specific for epitopes inaccessible in normal cells but accessible in metastatic cells, antibodies are selected whose binding decreases in normal cells with increasing cell density but whose binding remains constant in metastatic cells, as shown in Fig. 4. After labeling with fluorescein-secondary antibodies, EphA2 expression is evaluated by using flow cytometry. The antibodies that best distinguish between normal and metastatic cells are selected. Specificity for EphA2 is confirmed via western blotting and immunoprecipitation studies. Antibodies exhibiting the best selectivity are then humanized using art recognized techniques.

### EXAMPLE 6

### Altered EphA2 Expression Through Transfection

To assess the consequences of EphA2 overexpression, MCF-10A cells were transfected with human EphA2 cDNA (EphA2) or a vector control (vector). After establishing cultures of MCF-10A cells with stable overexpression of EphA2, microscopic evaluation revealed differences in the cell morphology as compared to vector-transfected control cells (not shown). Non-transformed MCF-10A cells display an epithelial morphology and interact with one-another, even at low cell density. In contrast, EphA2-overexpressing MCF-10A cells (MCF^{EphA2}) adopt a fibroblast-like morphology and do not form cell-cell contacts, even at high cell density. To confirm that the mesenchymal morphology does not represent clonal variation, a separate sample of MCF-10A cells transfected with EphA2 cDNAs yielded identical results.

Cell-ECM adhesions were evaluated by incubating cells on ECM at 37°C for 30 minutes before vigorous washing to remove weakly adherent cells. These assays revealed a 24-fold increase in ECM attachments in MCF^{EphA2} cells relative to vector-transfected controls (P < 4x10⁻⁴). Cell-cell adhesions were assayed by incubating cells in suspension and counting the average size of cell colonies. Whereas vector-transfected MCF-10A cells interact with one-another in colonies with an average size of 4.1 cells, the average colony size of MCF^{EphA2} cells is reduced to 1.3 cells (P < 3x10⁻⁵).

Since stable cell-cell contacts cause EphA2 to become enriched within sites of cell-cell contact, EphA2 subcellular localization was assessed by immunostaining with specific antibodies. The EphA2 on non-transformed MCF-10A cells was restricted to a narrow line where adjacent cells came into direct contact, with little staining of membrane that was not in contact with neighboring cells. In contrast, the pattern of EphA2 staining on MCF^{EphA2} cells was diffuse, with little staining of cell-cell contacts.

The lack of EphA2 within cell-cell contacts in MCF^{EphA2} cells was intriguing since EphA2 is stimulated by ligands that are anchored to the cell membrane. To measure EphA2 stimulation, the phosphotyrosine content of immunoprecipitated EphA2 was measured by Western blot analysis with phosphotyrosine specific antibodies. Whereas the EphA2 in vector-transfected MCF-10 cells was tyrosine phosphorylated, EphA2 was not tyrosine phosphorylated in MCF^{EphA2} cells. The decreased phosphotyrosine content was confirmed using multiple EphA2 antibodies for immunoprecipitation (D7, B2D6) and different phosphotyrosine-specific antibodies (4G10, PY20) for Western blot analyses.

### EXAMPLE 7

### Malignancy and Metastasis Through EphA2 Transfection

Malignant transformation was studied *in vitro,* and MCF^{EphA2} cells were found to colonize soft agar. Whereas vector-transfected MCF-10A cells formed 0.3 colonies per high-power field, while MCF^{EphA2} cells displayed increased colony growth in soft agar, with an average of 3.0 colonies per high-power field (P < 3x10⁻⁷). Vector and EphA2 overexpressing MCF-10A cells were allowed to interact with Matrigel (Collaborative, Bedford, MA). Non-transformed MCF-10A cells rapidly organized into spherical colonies when cultured on Matrigel whereas MCF^{EphA2} cells adopted a stellate organization that was indistinguishable from the behavior of metastatic cells (e.g., MDA-MB-231, MDA-MB,435).

Since *in vitro* analyses of transformation do not always predict tumorigenic potential *in vivo,* control or EphA2-overexpressing MCF-10A cells were implanted into athymic (*nu*/*nu*) mice. Subcutaneous injection of MCF^{EphA2} cells caused the formation of palpable tumors within four days in 19 of 19 mice. The median volume of resulting tumors related to the number of implanted cells and reached an average of 300 mm³ (for samples injected with 5x10⁶ cells) within 10 days (Table I). Necropsy revealed that the tumors were firmly attached to the underlying axillary muscle and surrounded by fibrous tissue. Histologically, the neoplastic cells were invasive and associated with fibrous connective tissue. These neoplastic cells exhibited moderate cytoplasmic and nuclear pleiomorphism and formed dysplastic tubular and secreting structures. In control experiments, cells transfected with vector DNA failed to grow in athymic mice (0 of 13; Table I) and necropsy failed to identify any growth or invasion of these cells.

Since the highest levels of EphA2 were consistently found in breast cancer cells that are metastatic *in vivo,* 1x10⁶ control or MCF^{EphA2} cells were injected into the tail vein of athymic mice. Within seven days, necropsy revealed lung micrometastases within large vessels in 2 of 4 mice injected with MCF^{EphA2} cells (Table I). The metastases were generally found to occlude large blood vessels but did not breach the vessel wall. Immunohistochemical staining with cytokeratin antibodies confirmed the epithelial nature of the thrombus and a lack of anti-thrombin staining revealed that the thrombus did not represent an abnormal or atypical outgrowth of endothelial cells. No lung colonization was observed in mice that had been injected with control MCF-10A cells (Table I).

**Table I. Tumorigenic and Metastatic Potential of EphA2-Transformed MCF-10A Cells**

| **Cell** | **Site of Inoculation** | **# of Cells Injected** | **Incidence of Tumorigenicity** | **Tumor Volume (mm**^{**3**}**)** |
|---|---|---|---|---|
| **Ctrl** | Subcutaneous | 1 x 10⁶ | 0/9 | N/A |
| ***EphA2*** | | 1 x 10⁶ | 9/9 | 66±20 |
| **Ctrl** | Subcutaneous | 5 x 10⁶ | 0/4 | N/A |
| ***EphA2*** | | 5 x 10⁶ | 10/10 | 293± 70 |
| **Ctrl** | Tail Vein | 1 x 10⁶ | 0/4 | |
| ***EphA2*** | | 1 x 10⁶ | 2/4 | |

### EXAMPLE 8

### Metastatic Targeting Using EphA2 Agonists

To test if EphA2 could be stimulated by an agonist, MCF^{EphA2} cells were suspended in soft agar in the presence or absence of 0.5 mg/mL EphrinA1-F_{c-}EphrinA1-F_{c} increased the phosphotyrosine content of EphA2, and EphrinA1-F_{c-}treated reduced colony formation in soft agar by 49% relative to vehicle-treated controls (P < 5x10⁻⁶). To test if EphA2 stimulation could alter cell behavior on Matrigel, the MCF^{EphA2} cells were treated with 0.5 mg/mL EphrinA1-F_{c}, which restored a spherical phenotype that was comparable to non-transformed MCF-10A cells. Thus, EphA2 stimulation reverses the effects of EphA2 overexpression. EphrinA1-F_{c}. Despite its inability to interact with its endogenous ligands, the EphA2 in MCF^{EphA2} cells responded to exogenous stimuli.

Although the invention has been described in detail with reference to preferred embodiments, variations and modifications exist within the scope and spirit of the invention as described and defined in the following claims.

Applicant's or agent's file reference number 290.0[?] [?]201 | International application N[?] PCT/US00/22670

INDICATIONS RELATING TO A DEPOSITED MICROORGANISM

(PCT Rule 13bis)

A. The indications made below relate to the microorganism referred to in the description
on page 6 , line 7 .

B. IDENTIFICATION OF DEPOSIT | Further deposits are identified on an additional sheet [X]

Name of depositary institution
American Type Culture Collection

Address of depositary institution (including postal code and country)
10801 University Boulevard
Manassas VA 20110-2209
United States of America

Date of deposit
8 December 2000 (08.12.00) | Accession Number
PTA 2755

C. ADDITIONAL INDICATIONS (leave blank if not applicable) | This information is continued on an additional sheet [ ]

Murine hybridoma D7

D. DESIGNATED STATES FOR WHICH INDICATIONS ARE MADE (if the indications are not for all designated States)

E. SEPARATE FURNISHING OF INDICATIONS (leave blank if not applicable)
The indications listed below will be submitted to the International Bureau later (specify the general nature of the indications e.g., "Accession Number of Deposit")

For receiving Office use only | For International Bureau use only
[ ] This sheet was received with the international application | [ ] This sheet was received by the International Bureau on:
Authorized officer | Authorized officer

Form PCT/RO/134 (July 1992) | LegalStar 1997, Form PCTMS

Applicant's or agent's file reference number 290.0[?] [?]0201 | International application No
PCT/US00/22670

INDICATIONS RELATING TO A DEPOSITED MICROORGANISM

(PCT Rule 13bis)

A. The indications made below relate to the microorganism referred to in the description
on page 6 , line 8 .

B. IDENTIFICATION OF DEPOSIT | Further deposits are identified on an additional sheet [ ]

Name of depositary institution
American Type Culture Collection

Address of depositary institution (including postal code and country)
10801 University Boulevard
Manassas VA 20110-2209
United States of America

Date of deposit | Accession Number
8 December 2000 (08.12.00) | PTA 2754

C. ADDITIONAL INDICATIONS (leave blank if not applicable) | This information is continued on an additional sheet [ ]

Murine hybridoma 82D6

D. DESIGNATED STATES FOR WHICH INDICATIONS ARE MADE (if the indications are not for all designated States)

E. SEPARATE FURNISHING OF INDICATIONS (leave blank if not applicable)
The indications listed below will be submitted to the International Bureau later (specify the general nature of the indications e.g., "Accession Number of Deposit")

For receiving Office use only | For International Bureau use only
[ ] This sheet was received with the international application | [ ] This sheet was received by the International Bureau on:
Authorized officer | Authorized officer

Form PCT/RO/134 (July 1992) | LegalStar 1997, Form PCTMS

## Claims

1. Use of an EphA2 agonist selected from (i) a compound comprising an extracellular domain of EphrinA1 and (ii) an antibody that is specific to an extracellular epitope of EphA2 having at least one biological activity for reducing metastatic invasion or proliferation of a tumor, for reducing metastasis of metastatic cells, for impeding proliferation of metastatic cells, for increasing the phosphotyrosine content of EphA2 in metastatic cells, and/or for reducing the invasiveness of metastatic cells compared to untreated metastatic cells for the preparation of a pharmaceutical composition for treatment of a mammalian metastatic tumor or cancer including a population of cells which overexpresses tyrosine kinase EphA2.

2. Use of an EphA2 agonist selected from (i) a compound comprising an extracellular domain of EphrinA1 and (ii) an antibody that is specific to an extracellular epitope of EphA2 for the manufacture of a medicament for the treatment of a metastatic tumor comprising a population of cells that overexpress EphA2.

3. Use of an EphA2 agonist selected from (i) a compound comprising an extracellular domain of EphrinA1 and (ii) an antibody that is specific to an extracellular epitope of EphA2 for the manufacture of a medicament for the treatment of a patient having a metastatic or potentially metastatic cancer comprising a population of cells that overexpress EphA2.

4. The use of any of the preceding claims, wherein the EphA2 agonist comprises an antibody.

5. The use of any of the preceding claims, wherein the EphA2 agonist comprises a monoclonal antibody.

6. The use of any of the preceding claims, wherein the EphA2 agonist comprises an antibody having specificity for an extracellular epitope of EphA2.

7. The use of any of the preceding claims, wherein the EphA2 agonist comprises an antibody that selectively binds to metastatic cells.

8. The use of any of the preceding claims, wherein the EphA2 agonist comprises an antibody conjugated to a cytotoxic agent.

9. The use of claim 8, wherein the cytotoxic agent is selected from the group consisting of a bacterial toxin, ricinA-chain, daunorubicin, methotrexate, a ribosome inhibitor, and a radioisotope.

10. The use of claim 9, wherein the cytotoxic agent is a radioisotope selected form the group consisting of an alpha emitter, a beta emitter, and an Auger electron emitter.

11. The use of any one of claims 1 to 3, wherein the EphA2 agonist comprises an ephrin that affects phosphorylation of EphA2.

12. The use of any one of claims 1 to 3, wherein the EphA2 agonist comprises a peptide.

13. The use of claim 12, wherein the EphA2 agonist comprises a peptide sequence defining an extracellular domain of EphrinA1.

14. The use of claim 13, wherein the peptide sequence is linked to a second peptide sequence defining immunoglobulin heavy chain.

15. The use of any one of claims 1 to 3, wherein the EphA2 agonist comprises an antisense oligonucleotide that affects EphA2 expression.

16. The use of any one of claims 1 to 3, wherein the population of cells forms at least a portion of a cancer tumor selected from the group consisting of breast, prostate, lung, and colon cancer tumors.

17. The use of any one of claims 1 to 3, wherein the population of cells comprises cells selected from the group consisting of breast cancer cells, prostate cancer cells, lung cancer cells and colon cancer cells.

18. The use of any one of claims 1 to 3, wherein the cells that overexpress EphA2 are epithelial cells.

## Patentansprüche

1. Verwendung eines EphA2-Agonisten, ausgewählt aus
(i) einer Verbindung, welche eine extrazelluläre Domäne von EphrinA1 umfasst, und
(ii) einem Antikörper, welcher spezifisch für ein extrazelluläres Epitop von EphA2 ist, mit mindestens einer biologischen Aktivität zum Reduzieren metastatischer Invasion oder von Proliferation eines Tumors, zum Reduzieren von Metastase von metastatischen Zellen, zum Verhindern von Proliferation von metastatischen Zellen, zum Erhöhen des Phosphotyrosin-Gehalts von EphA2 in metastatischen Zellen und/oder zum Reduzieren der invasiven Eigenschaft metastatischer Zellen im Vergleich zu unbehandelten metastatischen Zellen
für die Herstellung eines Arzneimittels zur Behandlung eines metastatischen Tumors oder Krebses bei einem Säuger einschließlich einer Population von Zellen, welche Tyrosinkinase EphA2 überexprimieren.

2. Verwendung eines EphA2-Agonisten, ausgewählt aus
(i) einer Verbindung, welche eine extrazelluläre Domäne von EphrinA1 umfasst, und
(ii) einem Antikörper, welcher spezifisch für ein extrazelluläres Epitop von EphA2 ist,
für die Herstellung eines Arzneimittels zur Behandlung eines metastatischen Tumors umfassend eine Population von Zellen, welche EphA2 überexprimieren.

3. Verwendung eines EphA2-Agonisten, ausgewählt aus
(i) einer Verbindung, welche eine extrazelluläre Domäne von EphrinA1 umfasst, und
(ii) einem Antikörper, welcher spezifisch für ein extrazelluläres Epitop von EphA2 ist,
für die Herstellung eines Arzneimittels zur Behandlung eines Patienten mit einem metastatischen oder potentiell metastatischen Krebs umfassend eine Population von Zellen, welche EphA2 überexprimieren.

4. Verwendung gemäß einem der vorangehenden Ansprüche, wobei der EphA2-Agonist einen Antikörper umfasst.

5. Verwendung gemäß einem der vorangehenden Ansprüche, wobei der EphA2-Agonist einen monoclonalen Antikörper umfasst.

6. Verwendung gemäß einem der vorangehenden Ansprüche, wobei der EphA2-Agonist einen Antikörper mit Spezifität für ein extrazelluläres Epitop von EphA2 umfasst.

7. Verwendung gemäß einem der vorangehenden Ansprüche, wobei der EphA2-Agonist einen Antikörper, der selektiv an metastatische Zellen bindet, umfasst.

8. Verwendung gemäß einem der vorangehenden Ansprüche, wobei der EphA2-Agonist einen Antikörper umfasst, der an ein cytotoxisches Agens konjugiert ist.

9. Verwendung gemäß Anspruch 8, wobei das cytotoxische Agens ausgewählt ist aus der Gruppe bestehend aus einem bakteriellen Toxin, einer RicinA-Kette, Daunorubicin, Methotrexat, einem Ribosomen-Inhibitor und einem Radioisotop.

10. Verwendung gemäß Anspruch 9, wobei das cytotoxische Agens ein Radioisotop ist, ausgewählt aus der Gruppe bestehend aus einem Alpha-Strahler, einem Beta-Strahler und einem Auger-Elektronen-Strahler.

11. Verwendung gemäß einem der Ansprüche 1 bis 3, wobei der EphA2-Agonist ein Ephrin umfasst, welches die Phosphorylierung von EphA2 beeinflusst.

12. Verwendung gemäß einem der Ansprüche 1 bis 3, wobei der EphA2-Agonist ein Peptid umfasst.

13. Verwendung gemäß Anspruch 12, wobei der EphA2-Agonist eine Peptidsequenz umfasst, welche eine extrazelluläre Domäne von EphrinA1 definiert.

14. Verwendung gemäß Anspruch 13, wobei die Peptidsequenz mit einer zweiten Peptidsequenz verknüpft ist, welche eine schwere Immunglobulin-Kette definiert.

15. Verwendung gemäß einem der Ansprüche 1 bis 3, wobei der EphA2-Agonist ein Antisense-Oligonucleotid umfasst, welches die EphA2-Expression beeinflusst.

16. Verwendung gemäß einem der Ansprüche 1 bis 3, wobei die Population an Zellen mindestens einen Teil eines Krebs-Tumors ausbildet, ausgewählt aus der Gruppe bestehend aus Brust-, Prostata-, Lungen-, und Dickdarm-Krebs-Tumoren.

17. Verwendung gemäß einem der Ansprüche 1 bis 3, wobei die Population der Zellen Zellen umfasst, die ausgewählt sind aus der Gruppe bestehend aus Brustkrebszellen, Prostatakrebszellen, Lungenkrebszellen und Dickdarmkrebszellen.

18. Verwendung gemäß einem der Ansprüche 1 bis 3, wobei die Zellen, welche EphA2 überexprimieren, epitheliale Zellen sind.

## Revendications

1. Utilisation d'un agoniste de EphA2 choisi parmi (i) un composé comprenant un domaine extracellulaire de l'EphrinAl et (ii) un anticorps qui est spécifique d'un épitope extracellulaire de EphA2, possédant au moins une activité biologique de réduction de l'invasion métastatique ou de la prolifération d'une tumeur, de réduction des métastases des cellules métastatiques, d'empêchement de la prolifération des cellules métastatiques, d'augmentation de la teneur en phosphotyrosine de EphA2 dans les cellules métastatiques, et/ou de réduction du pouvoir invasif des cellules métastatiques comparées à des cellules métastatiques non traitées, pour la préparation d'une composition pharmaceutique pour le traitement d'une tumeur ou d'un cancer métastatique de mammifère incluant une population de cellules qui surexpriment la tyrosine kinase EphA2.

2. Utilisation d'un agoniste de EphA2 choisi parmi (i) un composé comprenant un domaine extracellulaire de l'EphrinA1 et (ii) un anticorps qui est spécifique d'un épitope extracellulaire de EphA2 pour la fabrication d'un médicament pour le traitement d'une tumeur métastatique comprenant une population de cellules qui surexpriment EphA2.

3. Utilisation d'un agoniste de EphA2 choisi pami (i) un composé comprenant un domaine extracellulaire de l'EphrinAl et (ii) un anticorps qui est spécifique d'un épitope extracellulaire de EphA2 pour la fabrication d'un médicament pour le traitement d'un patient ayant un cancer métastatique, ou potentiellement métastatique, comprenant une population de cellules qui surexpriment EphA2.

4. Utilisation selon l'une quelconque des revendications précédentes, dans laquelle l'agoniste de EphA2 comprend un anticorps.

5. Utilisation selon l'une quelconque des revendications précédentes, dans laquelle l'agoniste de EphA2 comprend un anticorps monoclonal.

6. Utilisation selon l'une quelconque des revendications précédentes, dans laquelle l'agoniste de EphA2 comprend un anticorps ayant une spécificité pour un épitope extracellulaire de EphA2.

7. Utilisation selon l'une quelconque des revendications précédentes, dans laquelle l'agoniste de EphA2 comprend un anticorps se liant sélectivement aux cellules métastatiques.

8. Utilisation selon l'une quelconque des revendications précédentes, dans laquelle l'agoniste de EphA2 comprend un anticorps conjugué à un agent cytotoxique.

9. Utilisation selon la revendication 8, dans laquelle l'agent cytotoxique est choisi dans le groupe constitué d'une toxine bactérienne, de la chaîne ricine A, de daunorubicine, du méthotrexate, d'un inhibiteur ribosomique, et d'un radioisotope.

10. Utilisation selon la revendication 9, dans laquelle l'agent cytotoxique est un radioisotope choisi dans le groupe constitué d'un émetteur alpha, un émetteur bêta, et un émetteur d'électron Auger.

11. Utilisation selon l'une quelconque des revendications 1 à 3, dans laquelle l'agoniste de EphA2 comprend une ephrine affectant la phosphorylation de EphA2.

12. Utilisation selon l'une quelconque des revendications 1 à 3, dans laquelle l'agoniste de EphA2 comprend un peptide.

13. Utilisation selon la revendication 12, dans laquelle l'agoniste de EphA2 comprend une séquence peptidique définissant un domaine extracellulaire de l'EphrinA1.

14. Utilisation selon la revendication 13, dans laquelle la séquence peptidique est liée à un deuxième peptide définissant une chaîne lourde d'immunoglobuline.

15. Utilisation selon l'une quelconque des revendications 1 à 3, dans laquelle l'agoniste de EphA2 comprend une oligonucléotide antisens affectant l'expression de EphA2.

16. Utilisation selon l'une quelconque des revendications 1 à 3, dans laquelle la population de cellules forme au moins une portion d'une tumeur cancéreuse choisie parmi les cancers du sein, de la prostate, du poumon et du colon.

17. Utilisation selon l'une quelconque des revendications 1 à 3, dans laquelle la population de cellules comprend des cellules choisies dans le groupe constitué de cellules de cancer du sein, de cellules de cancer de la prostate, de cellules de cancer du poumon et de cellules de cancer du colon.

18. Utilisation selon l'une quelconque des revendications 1 à 3, dans laquelle les cellules qui surexpriment EphA2 sont des cellules épithéliales.
